# EUROPEAN PATENT SPECIFICATION

(11) **EP 0 751 760 B1**
(45) Date of publication and mention of the grant of the patent: **25.07.2001**
(21) Application number: 93901140.9
(22) Date of filing: 21.12.1992
(51) Int. Cl.: A61K 7/42, A61K 31/19, A61K 9/10, A61K 7/48

(54) **TREATMENT OF ULTRAVIOLET RADIATION-INDUCED ERYTHEMA**
BEHANDLUNG EINES DURCH UV-STRAHLUNG VERURSACHTEN ERYTHEMS
TRAITEMENT DE L'ERYTHEME PROVOQUE PAR UV

(30) Priority: 11.09.1992 US 943935
(43) Date of publication of application: 08.01.1997
(73) Proprietor: PERRICONE, Nicholas V., Dr., Guilford, Connecticut 06437 (US)
(72) Inventor: PERRICONE, Nicholas V., Dr., Guilford, Connecticut 06437 (US)
(74) Representative: Pendlebury, Anthony
(86) International application number: US9211037
(87) International publication number: WO9406405

(56) References cited:
- EP-A- 0 245 669
- EP-A- 0 261 812
- EP-A- 0 273 202
- EP-A- 0 293 579
- EP-A- 0 313 303
- EP-A- 0 456 459
- EP-A- 0 508 324
- US-A- 4 246 261
- US-A- 5 091 171
- DATABASE WPI Week 9215 Derwent Publications Ltd., London, GB; AN 92-117882 XP002016261 & JP-A-04 049 212 (SANSHO PHARM.KK)
- PHARMACEUTICAL FORMULAS, issued 1953, "The Chemist and Druggist, Glycerina, Glycerita, Vel Glycerola", see pg. 282 and 980.

## Description

The present invention relates to the topical application to the skin of active agents, and/or preparations containing them, for the treatment of erythema arising from radiation damage to the skin, and particularly for the treatment of skin which has received excessive exposure to ultraviolet radiation, as in the case of sunburn.

Ultraviolet induced burning of the skin is most commonly seen in persons who have been excessively exposed to natural sunlight (i.e., sunburn), but also can be seen in persons who have been excessively exposed to ultraviolet radiation from non-sunlight (artificial) sources, as may occur in tanning booths or incident to application of ultraviolet radiation as part of a therapeutic treatment. Cutaneous burn and other forms of skin damage also can arise from excessive or prolonged exposure to other forms of radiation outside the ultraviolet spectrum.

The clinical manifestations of ultraviolet induced burn seen in acute reactions is attributed to ultraviolet radiation in the range of 290 - 320 nanometers, generally designated ultraviolet B (UVB) radiation, although prolonged exposure to longer wavelength ultraviolet A light (-320 - 400 nanometers) can produce mild burn and marked hyperpigmentation.

The sunburn reaction is a complex inflammatory process causing dyskeratotic cells, spongiosis, vacuolation of kertinocytes and edema from capillary leakage, 12 to 24 hours after exposure to light. In addition to redness and pain, blisters may evolve. Chronic effects of UV light include degenerative changes of the skin which can lead to premalignant and malignant growths of the skin and degeneration of the dermal collagen.

The detrimental effects of sunburn have been postulated to be related to a transfer of energy from ultraviolet radiation to the skin, resulting in generation of excited oxygen species, such as singlet oxygen, the superoxide anion, and hydroxyl radicals that can damage lipid-rich membranes with the subsequent activation of the chemical mediators of inflammation. It is well known that ultraviolet B radiation releases arachidonic acid, which is quickly oxidized to a variety of biologically active metabolites, such as prostaglandins PGD2, PGE2, PGEF2. When arachadonic acid is oxidized via the cyclo-oxygenase pathway, prostaglandins create marked erythema. Arachadonic acid oxidized via the 5-lipo-oxygenase pathway produces leukotrienes, which also can cause erythema and edema. The free radicals created by ultraviolet radiation can also damage the DNA of the cells, resulting in permanent injury, premature aging, and carcinogenesis.

The clinical symptoms of ultraviolet burn are on a spectrum from mild increased sensitivity of the skin to severe pain. It should also be noted that damage to skin can be caused by other forms of radiation, that is, ionizing radiation as well as longer wave length radiation such as infrared, which can result in erythema and pigmentation as well as premature aging and malignancy. These other forms of radiation create damage by the same mechanism, i.e., generation of free radicals with subsequent damage to the cell membrane and DNA.

Suggestions for dealing with sunburn and other forms of ultraviolet radiation burn have predominantly been aimed at prevention rather than treatment. Several topical sunscreens have been developed, and have proved to be very effective in preventing sunburn and long-term photo-damage. However, these agents must be used prior to ultraviolet exposure in order to exert their protective effect.

EP-A-0508324 discloses the use of topical compositions containing a 2-hydroxycarboxylic acid and related compounds for use in alleviating or improving the dermatological signs of aging, including effects of sunlight or radiation which cause skin aging (such as thinning of the skin; deepening of skin lines; blemishes; blotches; nodules; atrophy; pigmented spots; precanerous lesions; elastotic changes characterized by leathery, coarse, rough, dry and yellowish skin; and telangiectactic skin).

The primary aim of this invention is to provide a treatment for radiation skin burn, particularly ultraviolet skin burn, and most particularly sunburn, and more particularly to provide a therapy based upon topical application to affected skin areas of an active agent or precursor thereof, preferably in association with a dermatologically acceptable carrier or vehicle.

The present invention provides the use of glycolic acid, a derivative of glycolic acid, or mixtures thereof for the preparation of a composition for topical administration for the treatment of ultraviolet radiation-induced erythema.

In the preferred practice of the invention, the glycolic acid (or derivative) is applied in admixture with a dermatologically acceptable carrier or vehicle (e.g., as a lotion, cream, ointment, soap, or the like) so as to facilitate topical application and, in some cases, provide additional therapeutic effects as might be brought about, e.g., by moisturizing of the affected skin areas.

The amount of the glycolic acid or derivative thereof necessary to bring about the therapeutic treatment of radiation-induced skin damage is not fixed per se, and necessarily is dependent upon the severity and extent of the condition, the form of glycolic acid employed, and the concentration of the glycolic acid when employed in association with a carrier. Generally, the glycolic acid or composition containing it is topically applied to the affected skin areas in a predetermined or as-needed regimen to bring about improvement, it generally being the case that gradual improvement is noted with each successive application. Insofar as has been determined based upon clinical studies to date, no adverse side effects are encountered.

This invention describes method for the treatment of radiation-induced erythema by the topical application of glycolic acids or glycolic acid derivatives to affected skin areas.

Glycolic acid is an "alpha-hydroxy acid", as used herein, the terminology "alpha-hydroxy acid" has reference to and encompasses the general class of organic compounds containing at least one hydroxy group and at least one carboxyl group, and wherein at least one hydroxyl group is located on the alpha-carbon atom. Typically, the compounds are organic acids having at least one carboxylic acid group and at least one hydroxyl group on the alpha-carbon atom, and may contain other functional groups including additional hydroxyl and carboxylic acid moieties. Most typically, alpha-hydroxy acids will have a basic structure of lower aliphatic compounds having from two to six carbon atoms.

The "derivatives" of the glycolic acid most typically will involve derivatives related to the carboxyl functionality, i.e., wherein the hydrogen or hydroxyl portion of the carboxyl moiety is substituted by metallic ions (to form salts), alkoxy groupings (to form esters), ammonium ions (to form ammonium salts), as well as other substitution reactions and products leading to formation of corresponding lactones, anhydrides or amines. However, the derivatives may also include reactions involving the alpha-hydroxyl group, most notably ketone formation, to form corresponding alpha-keto carboxylic acids.

Among the hydroxy acids and derivative compounds useful in the present invention are glycolic acid and hydroxymethylglycolic acid,

Glycolic acid and its derivatives have varying degrees of solubility in water, organic solvents, oils and the like. Since topical application to affected sites of radiation-induced erythema according to the invention requires that the active ingredient be in a form permitting such use, it generally will be the case that the glycolic acid or precursor or derivative thereof be employed in association with a carrier, and particularly one in which the active ingredient is soluble per se or is effectively solubilized (e.g., as an emulsion or microemulsion). It is necessary that the carrier be inert in the sense of not bringing about a deactivation of the hydroxy acid or derivative, and in the sense of not bringing about any adverse effect on the skin areas to which it is applied.

Suitable carriers include water, alcohols, oils and the like, chosen for their ability to dissolve or disperse the active ingredient at concentrations of active ingredient most suitable for use in the therapeutic treatment. Generally, even low concentrations of active ingredient in a carrier will be suitable, requiring only that more frequent topical application be resorted to. As a practical matter, however, to avoid the need for repeated application, it is desirable that the topically applied composition (i.e., glycolic acid or derivative plus carrier) be formulated to contain at least 0.5% by weight, more preferably at least 2% by weight, and most preferably at least 8 to 10% by weight, of the active ingredient, and accordingly, carriers will be chosen which can solubilize or disperse the active ingredient at such concentrations.

While the carrier for the glycolic acid or derivative of glycolic acid can consist of a relatively simple solvent or dispersant such as water or oils, it is generally preferred that the carrier comprise a composition more conducive to topical application, and particularly one which will form a film or layer on the skin to which it is applied so as to localize the application and provide some resistance to washing off by immersion in water or by perspiration and/or aid in the percutaneous delivery of the active agent. Many such compositions are known in the art, and can take the form of lotions, creams, gels or even solid compositions (e.g., stick-form preparations). Typical compositions include lotions containing water and/or alcohols and emollients such as hydrocarbon oils and waxes, silicone oils, vegetable, animal or marine fats or oils, glyceride derivatives, fatty acids or fatty acid esters or alcohols or alcohol ethers, lecithin, lanolin and derivatives, polyhydric alcohols or esters, wax esters, sterols, phospholipids and the like, and generally also emulsifiers (nonionic, cationic or anionic), although some of the emollients inherently possess emulsifying properties. These same general ingredients can be formulated into a cream rather than a lotion, or into gels, or into solid sticks by utilization of different proportions of the ingredients and/or by inclusion of thickening agents such as gums or other forms of hydrophillic colloids. Such compositions are referred to herein as dermatologically acceptable carriers.

As noted, the glycolic acid is provided in the form of the acid *per se* or in the form of a derivative. Exemplary compounds include the simple ionic salts (e.g., for glycolic acid, sodium glycolate, calcium glycolate, potassium glycolate, magnesium glycolate), fatty acid esters (e.g., lauryl glycolate, myristyl glycolate, palmityl glycolate, stearyl glycolate), anhydrides, or other chemical derivatives which preserve or provide the effective acid compound upon dissolution or dispersion in a carrier or upon topical application.

In order to demonstrate the efficacy of glycolic acid in treatment of radiation skin burn, a composition containing about 8% glycolic acid in an oil-in-water cream was prepared. Five test subjects were employed in the study, using their forearms as test sights. The test sights were covered with paper, with two windows 3 cm in diameter exposed on each forearm. These were then irradiated using a high pressure mercury halide lamp with 3 times the erythema dose (MED) of UVB irradiation. Upon the appearance of erythema (6 to 10 hours after irradiation), the glycolic acid composition was applied to one lesion of each forearm, and a control oil-in-water cream containing no glycolic acid was applied to the other. After 24 hours from the time of application, erythema in the treated areas had decreased as compared to the control areas to which only the oil-in-water cream (containing no glycolic acid) had been applied. After 72 hours, the glycolic acid-treated areas exhibited only hyperpigmentation, whereas the control areas exhibited marked erythema as well. The subjects all reported marked reduction in symptoms of burn on the areas treated with active ingredient.

Use of glycolic acid solubilized in an oil-in-water cream form is greatly preferred in the method of this invention for a variety of reasons. Glycolic acid is a small molecule, generally permeable to plasma membranes. In this carrier, the acid is better able to penetrate the skin, allowing the active ingredient to be released in a fat-soluble environment. Also, in this form the glycolic acid can be solubilized in a fat-soluble base (cream, lotion, etc.) which aids in skin penetration. Still further, this form of glycolic acid is less prone to oxidative breakdown (and particularly so when further admixed in a fat-soluble base), and thus is capable of being made up as a stable preparation having a suitably long shelf-life and which has prolonged effectiveness after being applied to the skin.

The mode by which the glycolic acid and derivatives are effective in treatment and prevention of sunburn as described herein is not particularly well understood, but may be related to the anti-oxidant properties of the active compounds and/or their interference with chemical reactions. It should also be noted that alpha-hydroxy acids and certain derivatives thereof have in the past been taught for preventing disorders of hyperkeratinization as well as other uses related to skin problems. See, for example, U.S. Pat. Nos. 3,920,835, 4,105,782, 4,197,316, 4,234,599, 4,380,549, and 5,091,171. While sunburn may have associated with it a component of hyperkeratosis, it is, if present, quite minimal in most cases as compared to other causes and effects of the problem, and accordingly the activity and effectiveness of the glycolic acid and derivatives in the present invention is not apparently related to effectiveness in treating hyperkeratinization disorders.

In terms of a possible explanation for the effectiveness of glycolic acid. in the treatment of radiation damage to the skin, it is noted that glycolic acid is an anti-oxidant which can scavenge free radicals such as the oxygen radicals created by exposure of cells to radiation, as well as the generation of free radicals produced by normal metabolism extracellularly and intracellularly. It is believed that glycolic acid sequesters metals, and may be involved in deactivation of the heme-containing dioxygenase that produces prostaglandin precursors in the endoplasmic reticulum. The plasma membranes of most cells are highly permeable to small-sized alpha-hydroxy acids and these may play a role in affecting the activity of prostaglandin production, e.g., by depressing prostaglandin cyclo-oxygenase where these compounds are employed in the method of the invention. Cyclo-oxygenase is a key enzyme in the oxidation of arachadonic acid, which leads the formation of prostaglandins which in turn mediates inflammation.

Having described the invention with reference to particular compositions, theories of effectiveness, and the like, it will be apparent to those of skill in the art that it is not intended that the invention be limited by such illustrative embodiments or mechanisms, and that modifications can be made without departing from the scope of the invention, as defined by the appended claims.

## Claims

1. Use of glycolic acid, a derivative of glycolic acid, or mixtures thereof for the preparation of a composition for topical administration for the treatment of ultraviolet radiation-induced erythema.

2. Use according to claim 1, wherein the derivative of glycolic acid is selected from the group consisting of ionic salts of glycolic acid and fatty acid esters or anhydrides of glycolic acid.

3. Use according to claim 1 or 2, wherein the active compound is provided in association with a dermatologically acceptable carrier.

4. Use according to claim 3, wherein the combination of the active compound and the carrier is in the form of a solution, dispersion, cream, lotion, gel or solid stick.

5. Use according to claim 4, wherein the carrier is an oil-in-water cream.

6. Use according to claim 4 or 5, wherein the combination comprises from 2 to 10% by weight glycolic acid.

7. Use according to claim 6, wherein the said combination comprises about 8% by weight glycolic acid.

## Patentansprüche

1. Verwendung von Glycolsäure, einem Derivat von Glycolsäure oder deren Mischungen für die Herstellung einer Zusammensetzung für eine topische Verabreichung zur Behandlung von durch Ultraviolett-Strahlung verursachtem Erythem.

2. Verwendung nach Anspruch 1, bei der das Derivat von Glycolsäure ausgewählt ist aus der Gruppe bestehend aus ionischen Salzen von Glycolsäure und Fettsäureestern oder -anhydriden von Glycolsäure.

3. Verwendung nach Anspruch 1 oder 2, bei der die aktive Verbindung in Verbindung mit einem dermatologisch annehmbaren Träger bereitgestellt wird.

4. Verwendung nach Anspruch 3, bei der die Kombination der aktiven Verbindung und des Trägers in Form einer Lösung, Dispersion, Creme, Lotion, eines Gels oder eines festen Stiftes vorliegt.

5. Verwendung nach Anspruch 4, bei der der Träger eine Öl-in-Wasser-Creme ist.

6. Verwendung nach Anspruch 4 oder 5, bei der die Kombination 2 bis 10 Gewichts-% Glycolsäure umfasst.

7. Verwendung nach Anspruch 6, bei der die Kombination etwa 8 Gewichts-% Glycolsäure umfasst.

## Revendications

1. Utilisation d'acide glycolique, d'un dérivé d'acide glycolique ou d'un de leurs mélanges pour la préparation d'une composition destinée à l'administration topique pour le traitement d'un érythème induit par les rayonnements ultraviolets.

2. Utilisation suivant la revendication 1, dans laquelle le dérivé d'acide glycolique est choisi dans le groupe consistant en des sels ioniques d'acide glycolique et des esters d'acides gras ou anhydrides d'acide glycolique.

3. Utilisation suivant la revendication 1 ou 2, dans laquelle le composé actif est fourni en association avec un support dermatologiquement acceptable.

4. Utilisation suivant la revendication 3, dans laquelle l'association du composé actif et du support est sous forme d'une solution, d'une dispersion, d'une crème, d'une lotion, d'un gel ou d'un bâtonnet solide.

5. Utilisation suivant la revendication 4, dans laquelle le support est une crème huile-dans-eau.

6. Utilisation suivant la revendication 4 ou 5, dans laquelle l'association comprend 2 à 10 % en poids d'acide glycolique.

7. Utilisation suivant la revendication 6, dans laquelle l'association comprend environ 8 % en poids d'acide glycolique.
